# EUROPEAN PATENT SPECIFICATION

(11) **EP 4 019 411 B1**
(45) Date of publication and mention of the grant of the patent: **29.01.2025**
(21) Application number: 20217119.5
(22) Date of filing: 23.12.2020
(51) Int. Cl.: B64D 45/00, B64D 13/06, A61B 5/00, A61B 5/0205

(54) **DATA-DRIVEN MANAGEMENT SYSTEM AND METHOD FOR PASSENGER SAFETY, HEALTH AND COMFORT**
DATENGESTEUERTES VERWALTUNGSSYSTEM UND VERFAHREN FÜR FAHRGASTSICHERHEIT, -GESUNDHEIT UND -KOMFORT
SYSTÈME ET PROCÉDÉ DE GESTION DE GUIDÉ PAR LES DONNÉES POUR LA SÉCURITÉ, LA SANTÉ ET LE CONFORT DE PASSAGERS

(43) Date of publication of application: 29.06.2022
(73) Proprietor: B/E Aerospace, Inc., Winston-Salem, NC 27105 (US)
(72) Inventor: ZHU, Yonghua, 01120 Montluel (FR); PEARSON, Matthew, Hartford, CT Connecticut 06106 (US)
(74) Representative: Dehns

(56) References cited:
- WO-A1-2016/197068
- DE-A1- 102017 109 730
- US-A1- 2013 338 857
- US-A1- 2017 066 534
- US-A1- 2019 061 772
- US-A1- 2020 031 474
- US-A1- 2020 079 524
- US-A1- 2020 104 616

## Description

### TECHNICAL FIELD

The present invention relates to a data-driven management system and method for passenger safety, health and comfort.

### BACKGROUND

Passenger cabins (e.g. train carriages, aircraft cabins, buses etc.) carry many passengers to/from target destinations. The well-being of the passengers during travel time in terms of safety, health and comfort is an important factor when passengers are travelling. Among all the systems, facilities and technologies for ensuring passengers' safety, health and comfort, particularly, the environmental control systems (ECS) play an important role. For example, in aircraft, the ECS can provide pressurized and conditioned fresh air to the aircraft cabin to ensure the health and comfort of the passengers and crew. However, and in the example of an aircraft, there are numerous complaints from passengers about the uncomfortable environment during the flight, which may include too dry, too hot, too cold, etc. In some examples, there are extreme cases where a person falls ill in a passenger cabin and no prompt help is provided by the crew of the passenger vehicle.

US 2013/338857 A1 discloses
a health management system for individual aircraft passengers. US 2017/066534 A1 discloses
a health, safety and comfort management system for individual aircraft passengers. US 2020/079524 A1 discloses
a temperature monitoring system for aircraft passenger rest compartments.

### SUMMARY OF THE INVENTION

In a first aspect, there is provided a management system according to claim 1.

The first set of parameters may be based on static data from the array of sensors.

The static data may include temperature, humidity, level of noise, amount of light in the cabin, air velocity and/or air quality.

The dynamic data may include activity levels of passenger, a perception of whether the passenger is too hot or too cold and/or an analysis of whether the passenger is showing symptoms of sickness.

The alert may be an audio alert and/or a visual alert.

In a second aspect, there is provided a method for passenger safety, health and comfort according to claim 6.

The first set of parameters may be based on static data from the array of sensors.

The static data may include temperature, humidity, level of noise, amount of light in the cabin, air velocity and/or air quality.

The dynamic data may include activity levels of passenger, a perception of whether the passenger is too hot or too cold and/or an analysis of whether the passenger is showing symptoms of sickness.

### BRIEF DESCRIPTION OF THE DRAWINGS

Figure 1 shows an example of a management system for passenger safety, health and comfort.
Figure 2 shows an example of a management method for passenger safety, health and comfort.

### DETAILED DESCRIPTION

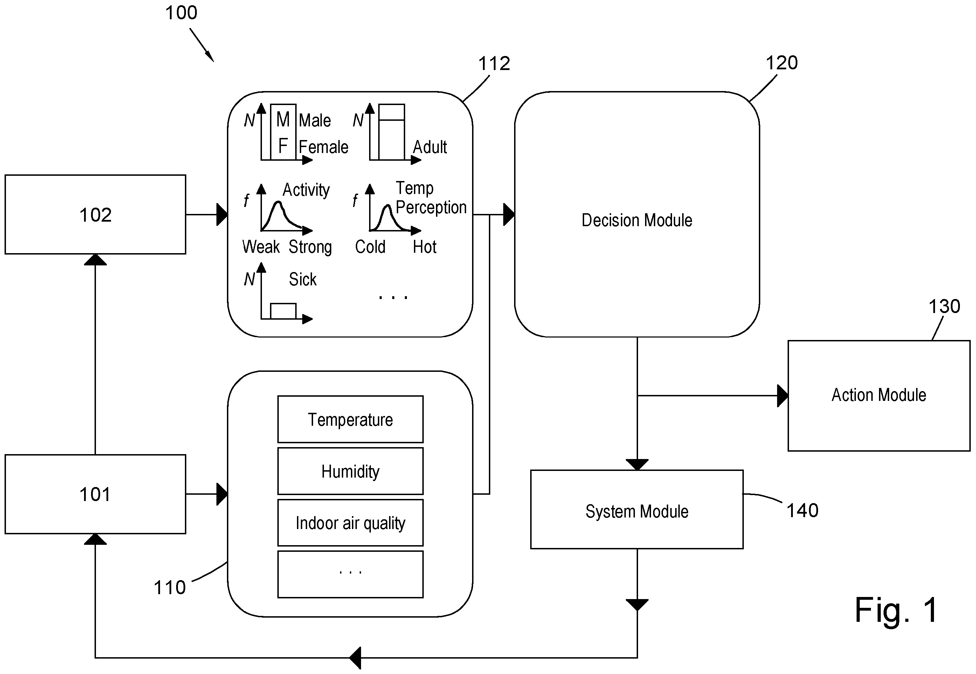

An example of a management system 100 for passenger safety, health and comfort is shown in Figure 1. The management system 100 includes a data acquisition module 101 that collects information from an array of sensors (not shown) within the passenger cabin. The array of sensors may include video sensing, image sensing, measurement sensing, soft sensing, and the like. The array of sensors are located within the passenger cabin and are utilised to sense various parameters of the environment. The data collected from the data acquisition module 101 may be filtered into data that has fixed values (e.g. static data) and data that needs further processing (e.g. dynamic data). The static data may include values that are provided to the data acquisition module from the environmental control system (ECS), or values from various sensors within the array of sensors in the passenger cabin. For example, the static data may include temperature, humidity and air quality of the air in the passenger cabin. The dynamic data that may need further processing, could include image processing and/or dynamic data retrieved from the various sensors in the array of sensors in the passenger cabin.

The data acquisition module 101 outputs a set of first parameters 110 based on the output of the static data. The first parameters may include, for example, the temperature, humidity, level of noise, amount of light in cabin, air velocity and air quality etc., of the passenger cabin. The data acquisition module 101 relays the dynamic data to an algorithm module 102. The algorithm module includes a processor that processes the dynamic data by image and data processing, integration, fusion and analysis to determine a second set of parameters 112 based on the outputs of the dynamic data after it has been processed. The second parameters include the gender of the passengers, whether the passengers are children or adults, estimated ages of the passengers. The second parameters may further include the activity levels of the passengers, a perception of whether the passengers are too hot or too cold, analysis of whether a passenger is showing symptoms of sickness, etc.

The first set of parameters 110 and the second set of parameters 112 are then output to a decision module 120. The decision module 120 includes one or more processors that process the information received from the first set of parameters 110 and the second set of parameters 120. The decision module 120 will sort through and analyse the data to determine the comfort level of the passengers and to determine if a passenger is showing symptoms of sickness. The comfort level of the passengers is, of course, based on various factors, such as the dryness of the air, humidity, light brightness in the cabin, temperature of the cabin, or the like.

The decision module 120 processes the data and determines if the passenger comfort levels are in the positive or in the negative. When the decision module 120 determines that the passenger comfort levels are in the positive, the decision module 120 determines that no further changes to the environment are necessary. When the decision module 120 determines that passenger comfort levels are in the negative, the decision module 120 determines the factors that are contributing to the level of discomfort by analysing the static and dynamic data. Based on this analysis, the decision module 120 determines what alterations need to be made to the cabin environment to raise the passenger comfort levels to the positive. In addition to the above, the decision module 120 may simultaneously determine if a passenger is showing symptoms of sickness by, for example, analysing the video/image sensors for signs of sickness. The decision module 120 would identify the passenger that is displaying symptoms and determine if the passenger is sick or is not sick.

The decision module 120 may then provide outputs based on the analysis of the data. The decision module 120 may filter alerts and actions that need to be taken to an action module 130 that may alert cabin staff to take necessary actions to ensure that the passenger comfort level is raised from negative to positive. Examples of such actions may be to alert cabin staff to provide blankets or water if the temperature is too low or the air is too dry. The decision module 120 may also alert cabin staff to take urgent measures if it is determined that a passenger is sick. Examples of these actions may be to provide medicine, oxygen or request an emergency to the passenger manager on board the vehicle. The alerts may be provided directly to the cabin staff by paging, pinging or alerting on a central computer or tablet - either with visual alerts and/or audible alerts.

In addition to the action module 130, there may be provided a system module 140 that runs additionally or simultaneously with the action module 130. The decision module 120, after processing the data, may output calculations to the system module 140 that do not need to be actioned by cabin staff and can directly be altered in the on-board computers to ensure that passenger comfort levels move from a negative to a positive. As an example, the decision module 120 may determine that the temperature of the cabin is too hot and, therefore, the decision module 120 would then output to the system module 140 that the temperature of the cabin needs to be reduced. The system module 140 then reduces the temperature of the cabin, where appropriate. Likewise, the decision module 120 may determine that the air of the cabin is too dry and, therefore, the decision module 120 instructs the system module 140 to change the humidity of the cabin air. Further examples may include, altering the brightness of the lights in response to the passenger comfort level being in the negative to raise the passenger comfort levels to the positive.

The system as described in Figure 1 therefore provides a data-driven management system that can ascertain if passengers are feeling comfortable and/or are experiencing symptoms of sickness. Therefore, the passenger well-being is monitored throughout the journey and necessary action is taken by the crew and/or by the environment control system of the passenger vehicle. The combination of static and dynamic data, as well as the processes described above, allow for a more efficient tackling of environmental factors that make the passengers more comfortable during their journey.

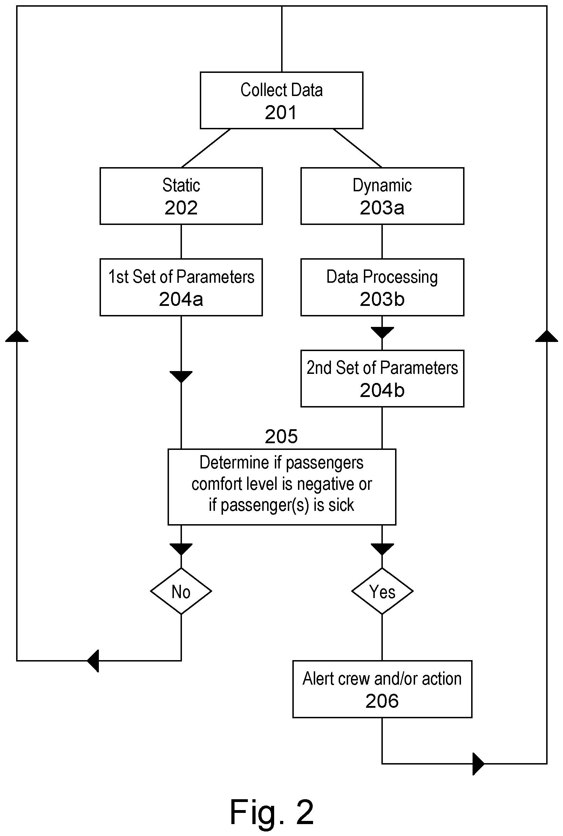

Figure 2 shows an example of a management method using the system described above for passenger safety, health and comfort.

At step 201, data is collected from the array of sensors as discussed above. The data collected at step 201 is then determined to be static data in step 202 or dynamic data in step 203a. The static data is then processed to determine a first set of parameters at step 204a. The dynamic data is separately processed and analysed in step 203b and then this information is processed to determine a second set of parameters at step 204b. At step 205, the first and second set of parameters are analysed to determine if the passengers comfort level is in the negative and whether a passenger is presenting symptoms of sickness. If it is determined that the passenger is uncomfortable and that the passenger comfort levels are in the negative and/or if a passenger is experiencing symptoms of sickness, the method alerts the crew of the cabin and/or provides instructions to the environmental control system to take action in the central computer (e.g. reduce temperature, increase humidity etc.) at step 206. After this step, the method returns to step 201 to continue monitoring of the cabin. If it is determined that the passenger comfort level is in the positive and/or that no passenger is experiencing symptoms of sickness, the method returns to step 201 to continue monitoring.

Although this disclosure has been described in terms of preferred examples, it should be understood that these examples are illustrative only and that the claims are not limited to those examples.

## Claims

1. A management system (100) for passenger safety, health and comfort, the system comprising:
a first module (101) configured to collect data from an array of sensors within an aircraft passenger cabin;
a first set of parameters (110) based on the data collected by the first module;
a second set of parameters (112) based on the data collected by the first module;
a second module (120) comprising a first processor configured to process the first set of parameters and the second set of parameters, wherein the processor is further configured to:
determine if passenger comfort levels are in the negative and if a passenger is showing symptoms of sickness;
determine alterations and/or actions to be taken by an environmental control system and/or crew of the aircraft passenger cabin to assist in moving the comfort levels to a positive and/or to assist a sick passenger; and
alert the crew and/or alter the environmental control system; wherein
the first module is a data acquisition module and the second module is a decision module; and
the management system further comprises an algorithm module, which is configured to receive dynamic data from the data acquisition module, and
**characterized in that**,
the algorithm module includes a second processor that processes the dynamic data by image and data processing, integration, fusion and/or analysis to determine the second set of parameters; and
the second set of parameters includes gender of passenger, age of passenger, and/or a determination of whether the passenger is an adult or a child.

2. The management system (100) of claim 1, wherein the first set of parameters is based on static data from the array of sensors.

3. The management system (100) of claim 2, wherein the static data includes temperature, humidity, level of noise, amount of light in the cabin, air velocity and/or air quality.

4. The management system (100) of claim 1 wherein the dynamic data further includes activity levels of passenger, a perception of whether the passenger is too hot or too cold and/or an analysis of whether the passenger is showing symptoms of sickness.

5. The management system of any preceding claim, wherein the alert is an audio alert and/or a visual alert.

6. A method for passenger safety, health and comfort, the method comprising:
collecting (201) data from an array of sensors within an aircraft passenger cabin with a first module (101);
determining (204a) a first set of parameters (110) based on the data collected by the first module;
determining (204b) a second set of parameters (112) based on the data collected by the first module;
analysing the first set of parameters and the second set of parameters with a second module (120) that includes a first processor, the method further comprising;
determining (205) if passenger comfort levels are in the negative and if a passenger is showing symptoms of sickness;
determining alterations and/or actions to be taken by an environmental control system and/or crew of the aircraft passenger cabin to assist in moving the comfort levels in the positive and/or to assist a sick passenger; and
alert (206) the crew and/or alter the environmental control system;
the management system further comprises an algorithm module (102) which is configured to receive dynamic data from the data acquisition module; and and
the first module is a data acquisition module and the second module is a decision module; **characterized in that**,
the second set of parameters includes gender of passenger, age of passenger, and/or a determination of whether the passenger is an adult or a child; and
**in that**
the algorithm module includes a second processor that processes the dynamic data by image and data processing, integration, fusion and/or analysis to determine the second set of parameters.

7. The method of claim 6, wherein the first set of parameters is based on static data from the array of sensors.

8. The method of claim 7, wherein the static data includes temperature, humidity, level of noise, amount of light in the cabin, air velocity and/or air quality.

9. The method of claim 8, wherein the dynamic data further includes activity levels of passenger, a perception of whether the passenger is too hot or too cold and/or an analysis of whether the passenger is showing symptoms of sickness.

## Patentansprüche

1. Verwaltungssystem (100) für Passagiersicherheit, -gesundheit und -komfort, wobei das System Folgendes umfasst:
ein erstes Modul (101), das dazu konfiguriert ist, Daten von einer Anordnung von Sensoren in einer Luftfahrzeugpassagierkabine zu sammeln;
einen ersten Parametersatz (110) basierend auf den durch das erste Modul gesammelten Daten;
einen zweiten Parametersatz (112) basierend auf den durch das erste Modul gesammelten Daten;
ein zweites Modul (120), das einen ersten Prozessor umfasst, der dazu konfiguriert ist, den ersten Parametersatz und den zweiten Parametersatz zu verarbeiten, wobei der Prozessor ferner zu Folgendem konfiguriert ist:
Bestimmen, ob sich die Passagierkomfortstufen negativ sind und ob ein Passagier Symptome von Krankheit zeigt;
Bestimmen von Änderungen und/oder Maßnahmen, die von einem Umweltsteuersystem und/oder einer Besatzung der Luftfahrzeugpassagierkabine zu ergreifen sind, um dabei zu unterstützen, die Komfortstufen ins Positive zu verschieben und/oder einen kranken Passagier zu unterstützen; und
Alarmieren der Besatzung und/oder Ändern des Umweltsteuersystems; wobei
das erste Modul ist ein Datenerfassungsmodul und das zweite Modul ist ein Entscheidungsmodul; und
das Verwaltungssystem ferner ein Algorithmusmodul umfasst, das dazu konfiguriert ist, dynamische Daten vom Datenerfassungsmodul zu empfangen, und
**dadurch gekennzeichnet, dass**
das Algorithmusmodul einen zweiten Prozessor umfasst, der die dynamischen Daten durch Bild- und Datenverarbeitung, Integration, Fusion und/oder Analyse verarbeitet, um den zweiten Parametersatz zu bestimmen; und
der zweite Parametersatz das Geschlecht eines Passagiers, das Alter eines Passagiers und/oder eine Bestimmung, ob der Passagier ein Erwachsener oder ein Kind ist, beinhaltet.

2. Verwaltungssystem (100) nach Anspruch 1, wobei der erste Parametersatz auf statischen Daten von der Anordnung von Sensoren basiert.

3. Verwaltungssystem (100) nach Anspruch 2, wobei die statischen Daten Temperatur, Luftfeuchtigkeit, Geräuschpegel, Lichtmenge in der Kabine, Luftgeschwindigkeit und/oder Luftqualität beinhalten.

4. Verwaltungssystem (100) nach Anspruch 1, wobei die dynamischen Daten ferner Aktivitätsstufen eines Passagiers, eine Wahrnehmung, ob dem Passagier zu heiß oder zu kalt ist, und/oder eine Analyse, ob der Passagier Krankheitssymptome zeigt, beinhalten.

5. Verwaltungssystem nach einem der vorhergehenden Ansprüche, wobei der Alarm ein akustischer Alarm und/oder visueller Alarm ist.

6. Verfahren für Passagiersicherheit, -gesundheit und -komfort, wobei das Verfahren Folgendes umfasst:
Sammeln (201) von Daten von einer Anordnung von Sensoren innerhalb einer Luftfahrzeugpassagierkabine mit einem ersten Modul (101);
Bestimmen (204a) eines ersten Parametersatzes (110) basierend auf den durch das erste Modul gesammelten Daten;
Bestimmen (204b) eines zweiten Parametersatzes (112) basierend auf den durch das erste Modul gesammelten Daten;
Analysieren des ersten Parametersatzes und des zweiten Parametersatzes mit einem zweiten Modul (120), das einen ersten Prozessor umfasst, wobei das Verfahren ferner Folgendes umfasst:
Bestimmen (205), ob sich die Passagierkomfortstufen negativ sind und ob ein Passagier Symptome von Krankheit zeigt;
Bestimmen von Änderungen und/oder Maßnahmen, die von einem Umweltsteuersystem und/oder einer Besatzung der Luftfahrzeugpassagierkabine zu ergreifen sind, um dabei zu unterstützen, die Komfortstufen ins Positive zu verschieben und/oder einen kranken Passagier zu unterstützen; und
Alarmieren (206) der Besatzung und/oder Ändern des Umweltsteuersystems;
wobei das Verwaltungssystem ferner ein Algorithmusmodul (102) umfasst, das dazu konfiguriert ist, dynamische Daten von dem Datenerfassungsmodul zu empfangen; und
das erste Modul ist ein Datenerfassungsmodul und das zweite Modul ist ein Entscheidungsmodul; **dadurch gekennzeichnet, dass** der zweite Parametersatz das Geschlecht eines Passagiers, das Alter eines Passagiers und/oder eine Bestimmung, ob der Passagier ein Erwachsener oder ein Kind ist, beinhaltet; und dadurch, dass
das Algorithmusmodul einen zweiten Prozessor umfasst, der die dynamischen Daten durch Bild- und Datenverarbeitung, Integration, Fusion und/oder Analyse verarbeitet, um den zweiten Parametersatz zu bestimmen.

7. Verfahren nach Anspruch 6, wobei der erste Parametersatz auf statischen Daten von der Anordnung von Sensoren basiert.

8. Verfahren nach Anspruch 7, wobei die statischen Daten Temperatur, Luftfeuchtigkeit, Geräuschpegel, Lichtmenge in der Kabine, Luftgeschwindigkeit und/oder Luftqualität beinhalten.

9. Verfahren nach Anspruch 8, wobei die dynamischen Daten ferner Aktivitätsstufen eines Passagiers, eine Wahrnehmung, ob dem Passagier zu heiß oder zu kalt ist, und/oder eine Analyse, ob der Passagier Krankheitssymptome zeigt, beinhalten.

## Revendications

1. Système de gestion (100) pour la sécurité, la santé et le confort des passagers, le système comprenant :
un premier module (101) configuré pour collecter des données à partir d'un ensemble de capteurs à l'intérieur d'une cabine passagers d'avion ;
un premier ensemble de paramètres (110) basé sur les données collectées par le premier module ;
un second ensemble de paramètres (112) basé sur les données collectées par le premier module ;
un second module (120) comprenant un premier processeur configuré pour traiter le premier ensemble de paramètres et le second ensemble de paramètres, dans lequel le processeur est également configuré pour :
déterminer si le niveau de confort des passagers est négatif et si un passager présente des symptômes de maladie ;
déterminer les modifications et/ou les mesures à prendre par un système de contrôle environnemental et/ou par l'équipage de la cabine passagers de l'aéronef pour aider à amener les niveaux de confort à un niveau positif et/ou pour aider un passager malade ; et
alerter l'équipage et/ou modifier le système de contrôle environnemental ; dans lequel
le premier module est un module d'acquisition de données et le second module est un module de décision ; et
le système de gestion comprend également un module d'algorithme, qui est configuré pour recevoir des données dynamiques du module d'acquisition de données, et
**caractérisé en ce que**,
le module d'algorithme comporte un second processeur qui traite les données dynamiques par traitement d'image et de données, intégration, fusion et/ou analyse pour déterminer le second ensemble de paramètres ; et
le second ensemble de paramètres comporte le sexe du passager, l'âge du passager et/ou la détermination du fait que le passager soit un adulte ou un enfant.

2. Système de gestion (100) selon la revendication 1, dans lequel le premier ensemble de paramètres est basé sur des données statiques provenant du réseau de capteurs.

3. Système de gestion (100) selon la revendication 2, dans lequel les données statiques comportent la température, l'humidité, le niveau de bruit, la quantité de lumière dans la cabine, la vitesse de l'air et/ou la qualité de l'air.

4. Système de gestion (100) selon la revendication 1, dans lequel les données dynamiques comportent également les niveaux d'activité du passager, une perception du fait que le passager a trop chaud ou trop froid et/ou une analyse du fait que le passager présente des symptômes de maladie.

5. Système de gestion selon une quelconque revendication précédente, dans lequel l'alerte est une alerte audio et/ou une alerte visuelle.

6. Procédé pour la sécurité, la santé et le confort des passagers, le procédé comprenant :
la collecte (201) des données à partir d'un ensemble de capteurs dans une cabine passagers d'aéronef avec un premier module (101) ;
la détermination (204a) d'un premier ensemble de paramètres (110) basé sur les données collectées par le premier module ;
la détermination (204b) d'un second ensemble de paramètres (112) basé sur les données collectées par le premier module ;
l'analyse du premier ensemble de paramètres et du second ensemble de paramètres avec un second module (120) qui comporte un premier processeur, le procédé comprenant également ;
la détermination (205) de l'éventualité que le niveau de confort des passagers est négatif et qu'un passager présente des symptômes de maladie ;
la détermination des modifications et/ou des mesures à prendre par un système de contrôle environnemental et/ou par l'équipage de la cabine passagers de l'aéronef pour aider à amener les niveaux de confort au niveau positif et/ou pour aider un passager malade ; et
l'alerte (206) de l'équipage et/ou la modification du système de contrôle environnemental ;
le système de gestion comprend également un module d'algorithme (102), qui est configuré pour recevoir des données dynamiques depuis le module d'acquisition de données ; et
le premier module est un module d'acquisition de données et le second module est un module de décision ; **caractérisé en ce que**, le second ensemble de paramètres comporte le sexe du passager, l'âge du passager et/ou la détermination du fait que le passager soit un adulte ou un enfant ; et **en ce que**
le module d'algorithme comporte un second processeur qui traite les données dynamiques par traitement d'image et de données, intégration, fusion et/ou analyse pour déterminer le second ensemble de paramètres.

7. Procédé selon la revendication 6, dans lequel le premier ensemble de paramètres est basé sur des données statiques provenant du réseau de capteurs.

8. Procédé selon la revendication 7, dans lequel les données statiques comportent la température, l'humidité, le niveau de bruit, la quantité de lumière dans la cabine, la vitesse de l'air et/ou la qualité de l'air.

9. Procédé selon la revendication 8, dans lequel les données dynamiques comportent également les niveaux d'activité du passager, une perception du fait que le passager a trop chaud ou trop froid et/ou une analyse du fait que le passager présente des symptômes de maladie.
